# EUROPEAN PATENT SPECIFICATION

(11) **EP 0 625 140 B1**
(45) Date of publication and mention of the grant of the patent: **11.12.1996**
(21) Application number: 92922190.1
(22) Date of filing: 27.10.1992
(51) Int. Cl.: C07C 275/64, A61K 31/17, C07C 323/44, C07C 259/06, C07D 295/15, A61K 31/16, A61K 31/40

(54) **CYCLOPROPYL N-HYDROXYUREA AND N-HYDROXYACETAMIDES WHICH INHIBIT LIPOXYGENASE**
CYCLOPROPYL-N-HYDROXYHARNSTOFF ALS LYPOXYGENASEINHIBITOREN
N-HYDROXYACETAMIDES ET N-HYDROXYUREE DE CYCLOPROPYLE INHIBANT LA LIPOXYGENASE

(30) Priority: 13.12.1991 JP 330485/91
(43) Date of publication of application: 23.11.1994
(73) Proprietor: PFIZER INC., New York, N.Y. 10017 (US)
(72) Inventor: IKEDA, Takafumi, Handa-shi, Aichi 475 (JP); KAWAI, Akiyoshi, Handa-shi, Aichi 475 (JP); MANO, Takashi, Handa-shi, Aichi 475 (JP); OKUMURA, Yoshiyuki, Chita-gun, Aichi 470-32 (JP); STEVENS, Rodney W., Handa-shi, Aichi 475 (JP)
(74) Representative: Moore, James William, Dr.
(86) International application number: US9208979
(87) International publication number: WO9312077

(56) References cited:
- EP-A- 0 436 199
- EP-A- 0 452 908
- WO-A-90/08545

## Description

This invention relates to novel cyclopropyl N-hydroxyurea and N-hydroxyacetamide derivatives. The compounds of the present invention inhibit the action of the enzyme lipoxygenase and are useful in the treatment or alleviation of inflammatory diseases, allergy and cardiovascular diseases in mammals. This invention also relates to pharmaceutical compositions comprising such compounds and to the use of such compounds in treating inflammatory diseases, allergy and cardiovascular diseases in mammals. This invention further relates to methods of making such compounds.

Arachidonic acid is known to be the biological precursor of several groups of endogenous metabolites, prostaglandins including prostacyclins, thromboxanes and leukotrienes. The first step of arachidonic acid metabolism is the release of arachidonic acid and related unsaturated fatty acids from membrane phospholipids, via the action of phospholipase. Free fatty acids are then metabolized either by cyclooxygenase to produce the prostaglandins and thromboxanes or by lipoxygenase to generate hydroperoxy fatty acids which may be further converted to the leukotrienes. Leukotrienes have been implicated in the pathophysiology of inflammatory diseases, including rheumatoid arthritis, gout, asthma, ischemia reperfusion injury, psoriasis and inflammatory bowel disease. Any drug that inhibits lipoxygenase is expected to provide significant new therapy for both acute and chronic inflammatory conditions.

Several review articles on lipoxygenase inhibitors have been reported (See H. Masamune et al., Ann. Rep. Med. Chem., **24**, 71-80 (1989) and B.J. Fitzsimmons et al., Leukotrienes and Lipoxygenases, 427-502 (1989).

Compounds of the same general class as the compounds of the present invention are disclosed in EP 279263 A2, EP 196184 A2, EP 436199 A, JP (Kohyo) 502179/88, JP (Appln.) 105048/90 and U.S. patent No. 4,822,809.

### SUMMARY OF THE INVENTION

The present invention provides novel cyclopropyl N-hydroxyurea and N-hydroxyacetamide derivatives of the following formula and their pharmaceutically acceptable salts. 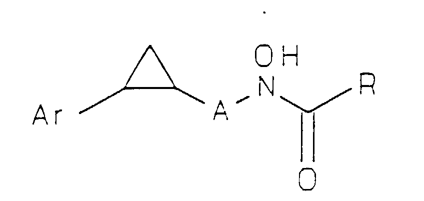 wherein A is C1 to C3 alkylene, Ar is phenyl or styryl, R is halosubstituted C1 to C3 alkyl, NHR' or 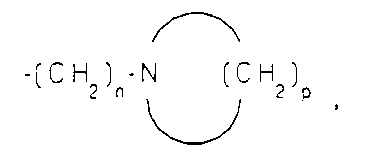 R' is C2 to C8 alkylthioalkyl, n is an integer of from 1 to 4 and p is an integer of from 2 to 5.

This invention also concerns pharmaceutical compositions comprising a pharmaceutically acceptable carrier or diluent and a compound of the invention or a pharmaceutically acceptable salt thereof. This invention further concerns methods of treating inflammatory diseases, allergy and cardiovascular diseases in mammals comprising administration of such compounds or compositions.

### DETAILED DESCRIPTION OF THE INVENTION

### Definitions

As used herein, the following definitions are used.

"Halo" and "halogen" mean radicals derived from the elements fluorine, chlorine, bromine and iodine.

"Alkyl" means straight or branched saturated hydrocarbon radicals, for example, methyl, ethyl, n-propyl and isopropyl.

"Halosubstituted alkyl' refers to an alkyl radical as described above substituted with one or more halogen radicals, for example, chloromethyl, bromoethyl and trifluoromethyl.

"Alkylene" means straight or branched alkene radicals, for example, methylene, 1,2-ethylene, 1,3-propylene and 1,2-propylene.

"Alkylthioelkyl" means a group of the structure -RSR wherein R is alkyl as defined above, for example, methylthiomethyl, methylthioethyl, methylthiopropyl, ethylthioethyl and propylthiomethyl.

This invention includes pharmaceutical compositions for treatment of inflammatory diseases, allergy and cardiovascular diseases in a mammal which comprises a pharmaceutically acceptable carrier or diluent and a compound of the above formula or a pharmaceutically acceptable salt thereof.

This invention also includes pharmaceutical compositions for inhibiting the lipoxygenase in a mammal which comprises a pharmaceutically acceptable carrier and a compound of the above formula or a pharmaceutically acceptable salt thereof.

The novel compounds of this invention may be prepared as shown in the reaction scheme described below.

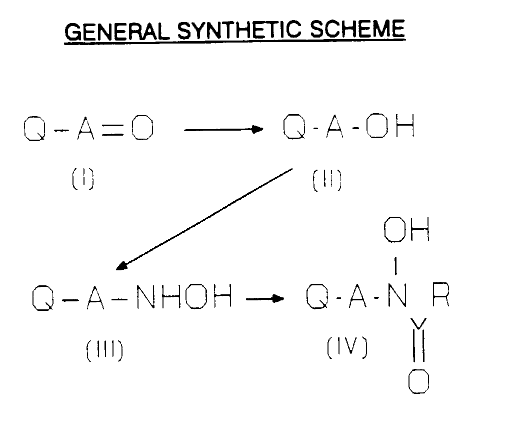

In the above scheme, A and R are as previously defined and Q is 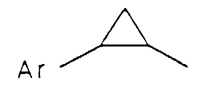 wherein Ar is phenyl or styryl.

The compounds of the invention are prepared according to the reaction steps explained in detail as follows.

The starting materials used in the procedure of the above reaction scheme may be prepared from commercially available compounds or known compounds according to standard methods known in the art.

In the first step, an alcohol (II) can be prepared via reduction of the corresponding ketone (I) with suitable reducing agent(s). Subsequently, the corresponding hydroxylamine (III) can be prepared by treating said alcohol with N,O-bis(tert-butoxycarbonyl)hydroxylamine under Mitsunobu-type reaction conditions followed by acid-catalyzed hydrolysis of the N,O-protected intermediate product (see JP (Kokai) 45344/89).

For example, ketone (I) is treated with tetrahydrofuran (THF) and a reducing agent (LiAIH₄, LiAIH(OC(CH₃)₃)₃ and the like) in a reaction-inert solvent. Preferred solvents include benzene, toluene, Et₂O, THF and methylene chloride. The reaction is usually carried out in the temperature range of from about -80° to about reflux temperature, with reaction times generally from several minutes to about 24 hours. The hydroxylamine thus obtained can be isolated by standard methods and purification can be achieved by conventional means, such as by recrystallization and chromatography.

The acetamides (IV) of the present invention can be prepared by treating the hydroxylamine (III) with a substituted acetyl chloride. For example, the hydroxylamine is reacted with the acetyl chloride in a reaction-inert solvent in the presence of a suitable base. Preferred bases include trimethylamine and pyridine; sodium hydride can also be used. Suitable solvents include methylene chloride, chloroform, THF, benzene and toluene. The reaction is usually carried out in the temperature range of from about 0°C to about room temperature, with reaction times generally from about 30 minutes to several hours. The final product acetamide (IV) can be isolated and purified by conventional means, such as by recrystallization and chromatography.

The ureas (IV) of the present invention can be prepared by treating the hydroxylamine (III) with a suitable isocyanate corresponding to the desired final product in a reaction-inert solvent. The reaction is usually carried out in the range of from room temperature through to reflux temperature. Suitable solvents which do not react with the hydroxylamine and/or isocyanate include, for example, THF, dioxane, methylene chloride and benzene. Preferred isocyanates include chloropropyl isocyanate, isocyanate propionic acid and trimethylsilyl isocyanate. The final product urea (IV) can be isolated and purified by conventional means, such as by recrystallization and chromatography.

The pharmaceutically acceptable salts of the novel compounds of the present invention are readily prepared by contacting said compounds with a stoichiometric amount of, in the case of a non-toxic cation, an appropriate metal hydroxide or alkoxide or amine in either aqueous solution or a suitable organic solvent, or, in the case of a non-toxic acid salt, an appropriate mineral or organic acid in either aqueous solution or a suitable organic solvent. The salt may then be obtained by precipitation or by evaporation of the solvent.

While the compounds of the present invention produced by the methods outlined above are racemic mixtures, they can be resolved into optically active isomers via known processes.

The compounds of this invention inhibit the activity of the enzyme lipoxygenase. This inhibition has been demonstrated by an assay using rat peritoneal cavity-resident cells which determines the effect of said compounds on the metabolism of arachidonic acid.

All of the compounds of Examples 1 to 4 were tested according to the methods described in "Synthesis of leukotrienes by peritoneal macrophages," Jap. J. Inflammation, 7, 145-150 (1987), and were shown to be lipoxygenase inhibitors, exhibiting IC₅₀ values in the range of about 0.186 to about 24.5 µM, for lipoxygenase inhibition.

The ability of the compounds of the present invention to inhibit lipoxygenase makes them useful for controlling the symptoms induced by the endogenous metabolites arising from arachidonic acid in a mammalian subject. The compounds are therefore valuable in the prevention and treatment of such disease states in which the accumulation of arachidonic acid metabolites are the causative factor, e.g., allergic bronchial asthma, skin disorders, rheumatoid arthritis, osteoarthritis and thrombosis.

The compounds of the invention and their pharmaceutically acceptable salts are of particular use in the treatment or alleviation of inflammatory diseases, allergy and cardiovascular diseases in a human subject as well in the inhibition of lipoxygenase.

### Methods of Administration

For treatment of the various conditions described above, the compounds of the invention and their pharmaceutically acceptable salts can be administered to a human subject either alone or, preferably, in combination with pharmaceutically acceptable carriers or diluents in a pharmaceutical composition, according to standard pharmaceutical practice. A compound can be administered via a variety of conventional routes of administration including orally, parenterally and by inhalation. When the compounds are administered orally, the dose range will generally be from about 0.1 to about 20 mg/kg/day, based on the body weight of the subject to be treated, preferably from about 0.1 to about 1.0 mg/kg/day in single or divided doses. If parenteral administration is desired, then an effective dose will generally be from about 0.1 to about 1.0 mg/kg/day. In some instances it may be necessary to use dosages outside these limits, since the dosage will necessarily vary according to the age, weight and response of the individual patient as well as the severity of the patient's symptoms and the potency of the particular compound being administered.

For oral administration, the compounds of the invention and their pharmaceutically acceptable salts can be administered, for example, in the form of tablets, powders, lozenges, syrups or capsules, or as an aqueous solution or suspension. In the case of tablets for oral use, carriers which are commonly used include lactose and com starch. Lubricating agents such as magnesium stearate are commonly added. In the case of capsules, useful diluents are lactose and dried com starch. When aqueous suspensions are required for oral use, the active ingredient is combined with emulsifying and suspending agents. If desired, certain sweetening and/or flavoring agents can be added.

For intramuscular, intraperitoneal, subcutaneous and intravenous use, a sterile solution of the active ingredient is usually prepared, and the pH of the solutions should be suitably adjusted and buffered. For intravenous use, the total concentration of solute should be controlled to make the preparation isotonic.

### Examples

The present invention is illustrated by the following examples. However, it should be understood that the invention is not limited to the specific details of these examples.

Proton nuclear magnetic resonance spectra (NMR) were measured at 270 MHz unless otherwise indicated and peak positions are expressed in parts per million (ppm) downfield from tetramethylsilane. The peak shapes are denoted as follows: s, singlet; d, doublet; t, triplet; q, quartet; m, multiplet; br, broad.

### Example 2 N-hydroxy-N'-(3-methythiopropyl)-N-[(trans-2-phenylcyclopropyl)methyl] urea

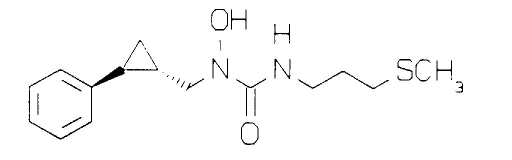

To a stirred solution of N'-(3-chloropropyl)-N-hydroxy-N-[(trans-2-phenylcyclopropyl)methyl]urea (0.79 g, 2.8 mM) in dry THF (10 ml) was added sodium thiomethoxide (0.5 g, 16.7 mM) at room temperature. After stirring ovemight, it was poured into saturated aqueous ammonium chloride. The whole was extracted with ethyl acetate (2 x 120 ml). The extracts were washed with water and brine, then were dried and evaporated in vacuo. The product was recrystallized from ethyl acetate/n-hexane, vacuum fitered and dried to fumish 0.82 g (35% yield) of the desired product, m.p. 103-104°C.
IR (neat): 3360, 1590, 1550, 1255, 1160, 1110, 750, 690.
NMR (DMSO-d6): 9.23 (s, 1H), 7.14 (m, 5H), 6.97 (t, J=6.2 Hz, 1H), 3.35 (m, 2H), 3.11 (q, J=6.6 Hz, 2H), 2.42 (t, J=6.6 Hz, 2H), 2.04 (s, 3H), 1.85 (m, 1H), 1.67 (m, 2H), 1.33 (m, 1H), 0.88 (m, 2H).

### Example 3 α-chloro-N-hydroxy-N-[(trans-2-phenylcyclopropyl)methyl]acetamide

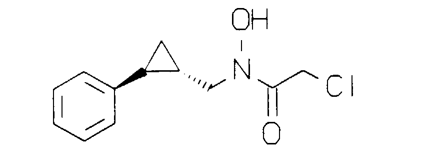

To a stirred solution of N-[(trans-2-phenylcyclopropyl)methyl]-N-hydroxylamine (3.26 g, 20 mM) in methylene chloride (50 ml) was added chloroacetyl chloride (4.52 g, 40 mM) and triethylamine (4.04 g, 40 ml) at room temperature. After stirring for 5 hours, it was poured into saturated aqueous sodium bicarbonate. The whole was extracted with chloroform (2 x 100 ml). The extracts were washed with water and brine, then were dried and evaporated in vacuo. Chromatographic purification (eluent chloroform:ethanol=15:1) of the residue provided 2 g (41.8% yield) of the desired product as a pale yellow oil.
IR (neat): 3200, 1640, 1500, 1245, 1175, 1090, 1030, 925.
NMR (DMSO-d6): 10.09 (s, 1H), 7.10 (m, 5H), 4.40 (s, 2H), 3.57 (d, J=7.0 Hz, 2H), 1.88 (m, 1H), 1.37 (m, 1H), 0.94 (m, 1H).

### Example 4 N-hydroxy-N-[(trans-2-phenyl-1-cyclopropyl)methyl]-α-(1-pyrrolidino)acetamide

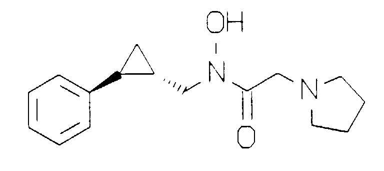

To a stirred solution of α-chloro-N-hydroxy-N-[(trans-2-phenylcyclopropyl)methyl]acetamide (0.75 g, 3.13 mM) in THF (7 ml) was added pyrrolidine (0.29 g, 4.07 mM) at room temperature. After stirring for 2 hours, volatiles were removed in vacuo. Ethyl acetate and saturated aqueous sodium bicarbonate was added to the residue and the whole was extracted with ethyl acetate. The extracts were washed with brine then dried and evaporated in vacuo. The resulting residue was triturated with diethylether and filtered to afford 0.532 g (62% yield) of the desired product as a colorless solid, m.p. 101-102.5°C.
IR (nujol): 1650, 1260, 1180, 1160, 1020, 880, 755, 700.
NMR (DMSO-d6): 9.79 (s, 1H), 7.18 (m, 4H), 3.52 (d, J=6.6 Hz, 2H), 3.36 (s, 2H), 2.50 (m, 4H), 1.88 (m, 1H), 1.66 (br s, 4H), 1.34 (m, 1H), 0.92 (m, 2H).

## Claims

1. A compound of the formula 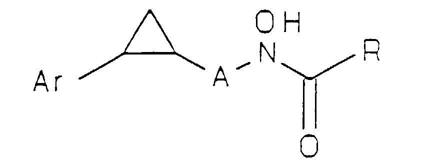 or a pharmaceutically acceptable salt thereof, wherein:
A is C1 to C3 alkylene;
Ar is phenyl or styryl;
R is halosubstituted C1 to C3 alkyl, NHR' or 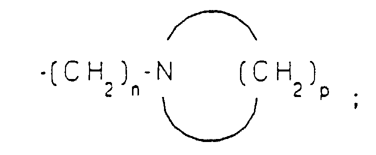
R' is C2 to C8 alkylthioalkyl;
n is an integer of from 1 to 4; and
p is an integer of from 2 to 5.

2. A compound according to Claim 1 wherein:
Ar is phenyl; and
R'is C2 to C8 alkylthioalkyl.

3. A compound according to Claim 1 wherein:
Ar is phenyl; and
R is halosubstituted C1 to C3 alkyl.

4. A compound according to Claim 1 wherein:
Ar is phenyl; and
R is pyrrolidinomethyl.

5. A compound according to Claim 1 selected from:
N-hydroxy-N'-(3-methylthiopropyl)-N-[(trans-2-phenylcyclopropyl)methyl]urea;
α-chloro-N-hydroxy-N-[(trans-2-phenylcyclopropyl)methyl]acetamide; and
N-hydroxy-N-[(trans-2-phenyl-1-cyclopropyl)methyl]-α-(1-pyrrolidino)acetamide.

6. A pharmaceutical composition comprising a compound according to any one of claims 1 to 5, or a pharmaceutically acceptable salt thereof, together with a pharmaceutically acceptable carrier.

7. A compound according to any one of claims 1 to 5, or a pharmaceutically acceptable salt thereof, or a pharmaceutical composition according to claim 6, for use as a medicament.

8. The use of a compound according to any one of claims 1 to 5, or a pharmaceutically acceptable salt thereof, or a pharmaceutical composition according to claim 6, for the manufacture of a medicament for inhibiting lipoxygenase in a mammal.

9. The use of a compound according to any one of claims 1 to 5, or a pharmaceutically acceptable salt thereof, or a pharmaceutical composition according to claim 6, for the manufacture of a medicament for the treatment of allergy or inflammatory or cardiovascular conditions in a mammal.

10. A process for the preparation of a compound according to claim 1 which comprises treating a compound of the formula 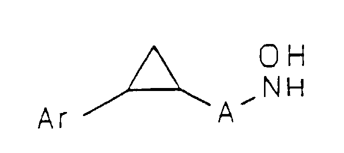 wherein A and Ar are as defined in claim 1 with either
(a) a substituted acetyl chloride in the presence of a suitable base in a reaction-inert solvent or
(b) a suitable isocyanate in a reaction-inert solvent
followed, in each case, by conventional isolation and purification of the final product and optional conversion, where appropriate, to a pharmaceutically acceptable salt.

## Patentansprüche

1. Verbindung der Formel 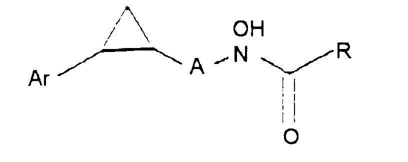 oder ein pharmazeutisch verträgliches Salz davon, worin:
A C1- bis C3-Alkylen darstellt;
Ar Phenyl oder Styryl darstellt;
R halogensubstituiertes C1- bis C3-Alkyl, NHR' oder 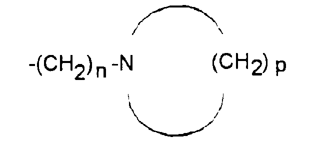 darstellt,
R' C2- bis C3-Alkylthioalkyl darstellt;
n eine ganze Zahl von 1 bis 4 ist; und
p eine ganze Zahl von 2 bis 5 ist.

2. Verbindung nach Anspruch 1, worin
Ar Phenyl darstellt; und
R' C2- bis C3-Alkylthioalkyl darstellt.

3. Verbindung nach Anspruch 1, worin
Ar Phenyl darstellt und
R halogensubstituiertes C1- bis C3-Alkyl darstellt.

4. Verbindung nach Anspruch 1, worin Ar Phenyl darstellt und R Pyrrolidinomethyl darstellt.

5. Verbindung nach Anspruch 1, ausgewählt aus N-Hydroxy-N'-(3-methylthiopropyl)-N-[(trans-2-phenylcyclopropyl)methyl]harnstoff; α-Chlor-N-hydroxy-N-[(trans-2-phenylcyclopropyl)methyl]acetamid und N-Hydroxy-N-[(trans-2-phenyl-1-cyclopropyl)methyl]-α-(1-pyrrolidino)-acetamid.

6. Pharmazeutische Zusammensetzung, umfassend eine Verbindung nach einem der Ansprüche 1 bis 5 oder ein pharmazeutisch verträgliches Salz davon, zusammen mit einem pharmazeutisch verträglichen Träger.

7. Verbindung nach einem der Ansprüche 1 bis 5 oder ein pharmazeutisch verträgliches Salz davon oder eine pharmazeutische Zusammensetzung nach Anspruch 6 zur Verwendung als Arzneimittel.

8. Verwendung einer Verbindung nach einem der Ansprüche 1 bis 5 oder eines pharmazeutisch verträglichen Salzes davon oder einer pharmazeutischen Zusammensetzung nach Anspruch 6 zur Herstellung eines Arzneimittels zur Inhibierung von Lipoxygenase bei einem Säuger.

9. Verwendung einer Verbindung nach einem der Ansprüche 1 bis 5 oder eines pharmazeutisch verträglichen Salzes davon oder einer pharmazeutisch verträglichen Zusammensetzung nach Anspruch 6 zur Herstellung eines Arzneimittels zur Behandlung von Allergie oder entzündlichen oder kardiovaskulären Zuständen bei einem Säuger.

10. Verfahren zur Herstellung einer Verbindung nach Anspruch 1, umfassend Behandeln einer Verbindung der Formel 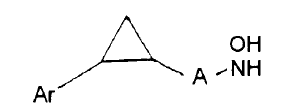 worin A und Ar wie in Anspruch 1 definiert sind, mit entweder
(a) einem substituierten Acetylchlorid in Gegenwart einer geeigneten Base in einem reaktionsinerten Lösungsmittel oder
(b) einem geeigneten Isocyanat in einem reaktionsinerten Lösungsmittel,
gefolgt in jedem Fall von üblicher Isolierung und Reinigung des Endprodukts und gegebenenfalls Umwandlung, falls geeignet, in ein pharmazeutisch verträgliches Salz.

## Revendications

1. Composé de formule 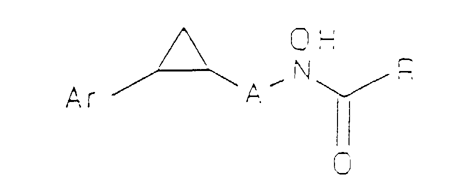 ou un de ses sels pharmaceutiquement acceptables, formule dans laquelle :
A représente un groupe alkylène en C₁ à C₃ ;
Ar représente un groupe phényle ou styryle ;
R représente un groupe alkyle en C₁ à C₃ halogénosubstitué, un groupe NHR' ou un groupe 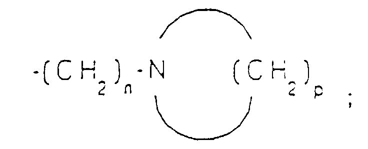
R' représente un groupe alkylthioalkyle en C₂ à C₈ ;
n est un nombre entier de 1 à 4 ; et
p est un nombre entier de 2 à 5.

2. Composé suivant la revendication 1, dans lequel :
Ar représente un groupe phényle ; et
R' représente un groupe alkylthioalkyle en C₂ à C₈.

3. Composé suivant la revendication 1, dans lequel :
Ar représente un groupe phényle ; et
R représente un groupe alkyle en C₁ à C₃ halogéno-substitué.

4. Composé suivant la revendication 1, dans lequel :
Ar représente un groupe phényle ; et
R représente un groupe pyrrolidinométhyle.

5. Composé suivant la revendication 1, choisi entre
le N-hydroxy-N'-(3-méthylthiopropyl)-N[(trans-2-phénylcyclopropyl)méthyl]urée ;
le α-chloro-N-hydroxy-N-[(trans-2-phénylcyclopropyl)méthyl]acétamide ; et
le N-hydroxy-N-[(trans-2-phényl-1-cyclopropyl)méthyl]-α-(1-pyrrolidino)acétamide.

6. Composition pharmaceutique comprenant un composé suivant l'une quelconque des revendications 1 à 5, ou un de ses sels pharmaceutiquement acceptables, en association avec un support pharmaceutiquement acceptable.

7. Composé suivant l'une quelconque des revendications 1 à 5, ou un de ses sels pharmaceutiquement acceptables, ou bien une composition pharmaceutique suivant la revendication 6, pour une utilisation comme médicament.

8. Utilisation d'un composé suivant l'une quelconque des revendications 1 à 5, ou d'un de ses sels pharmaceutiquement acceptables, ou bien d'une composition pharmaceutique suivant la revendication 6 pour la production d'un médicament destiné à inhiber la lipoxygénase chez un mammifère.

9. Utilisation d'un composé suivant l'une quelconque des revendications 1 à 5, ou d'un de ses sels pharmaceutiquement acceptables, ou bien d'une composition pharmaceutique suivant la revendication 6, pour la production d'un médicament destiné au traitement de l'allergie ou d'affections inflammatoires ou cardiovasculaires chez un mammifère.

10. Procédé pour la préparation d'un composé suivant la revendication 1, qui comprend le traitement d'un composé de formule 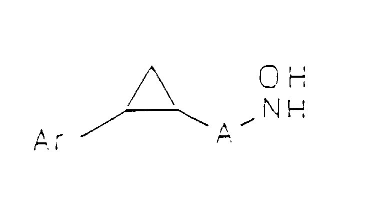 dans laquelle A et Ar répondent aux définitions suivant la revendication 1 avec
(a) un chlorure d'acétyle substitué en présence d'une base convenable dans un solvant inerte vis-à-vis de la réaction, ou bien
(b) un isocyanate convenable dans un solvant inerte vis-à-vis de la réaction,
puis, dans chaque cas, un isolement et une purification classiques du produit final et une transformation facultative, lorsque cela est approprié, en un sel pharmaceutiquement acceptable.
